# EUROPEAN PATENT APPLICATION

(11) **EP 4 400 125 A1**
(43) Date of publication of application: **17.07.2024**
(21) Application number: 22867731.6
(22) Date of filing: 08.09.2022
(51) Int. Cl.: A61L 27/52, A61L 27/18, A61B 17/56

(54) **SOL-GEL TRANSITION OF 6-ARM PEG HYDROGEL OVER TIME**

(30) Priority: 09.09.2021 KR 20210120387
(71) Applicant: Sunbio Inc., Gunpo-si, Gyeonggi-do 15849 (KR)
(72) Inventor: HWANG, Seongu, Goyang-si Gyeonggi-do 10374 (KR); KIM, Ga Eun, Uiwang-si Gyeonggi-do 16057 (KR)
(74) Representative: Plasseraud IP
(86) International application number: PCT/KR2022/013531
(87) International publication number: WO 2023/038463

(57) **Abstract**

The present invention relates to a 6-arm PEG hydrogel composition whose physical properties are converted from a sol to a gel within 30 minutes over time.

In the present invention, two types of PEG derivatives are included as a main component of the hydrogel, and when each of the PEG derivatives is dissolved in a buffer solution, and then the two solutions are mixed, it can be converted in physical properties to a gel state within 30 minutes.

## Description

### [Technical Field]

The present invention relates to a PEG hydrogel whose physical properties are converted from a sol to a gel over time.

### [Background Art]

Osteoarthritis is a joint disease caused by bone exposure due to wear of the articular cartilage surrounding the articular surface of the bone, pain due to inflammation of the synovial membrane that wraps around and connects the joint bones, and structural deformation and degeneration of the joint. This mainly influences joints on which body weight is loaded which causes pain, restricts activity, and causes body deformation, and can be caused by genetic predisposition, main joint trauma, occupational repetitive joint use, obesity, etc. In particular, it is a common disease among the elderly and is gradually increasing with the global aging trend. Until now, the goal in treatment of such osteoarthritis has not been to repair structural damage to the joint, but to focus on symptom relief, pain reduction, and delaying disease progression to maintain joint function and quality of life. As one treatment for osteoarthritis, intra-articular injection therapy is being performed, representative examples of which are steroid injection and hyaluronic acid injection. Steroid injection shows a temporary analgesic effect of 80 to 90%, but frequent injections have been reported to worsen joint damage, so that it is recommended that the injections be performed 4 to 6 months apart. Hyaluronic acid injection has been attempted as a way to supplement hyaluronic acid depleted by the inflammatory response of osteoarthritis from the outside, and is called a viscosupplement.

The history of hyaluronic acid injection therapy (Viscosupplementation) started with veterinary medicine for racehorses in the 1970s, and products such as Healon^{®} and Hylartil-Vet^{®} were developed and marketed. Then, in 1987, Artz^{®} (from Seikagaku, Japan) and Hyalgan^{®} (from FIDIA, Italy) were manufactured for the treatment of arthritis patients. Synvisc^{®} was developed by Balazs et al. in the 1990s as a result of steady R&D using hyaluronic acid, and due to the sales of hyaluronic acid products for single injection using various low molecular weights, it has since been widely used in a supplementary therapy to delay surgery for arthritis patients in Japan, and improved products have been developed and marketed all over the world, including Korea and Europe (Non-Patent Document 1).

Hyaluronic acid is a natural component of the extracellular matrix, and is a linear polymeric polysaccharide in which β-D-N-acetylglucosamine and β-D-glucuronic acid are alternately combined. When injected intraarticularly, it acts as a lubricant and shock absorber to relieve the pain of osteoarthritis and improve knee function. The molecular weight of hyaluronic acid in the synovial fluid of a healthy young person is 6,000,000 Da, and the dynamic analysis values (at 2.5 Hz) are about 45 Pa in viscosity and 117 Pa in elasticity (Non-Patent Document 2). Currently, commercially available hyaluronic acid injection products can be divided into products consisting of linear hyaluronic acid itself and products consisting of a hyaluronic acid gel form by crosslinking hyaluronic acid. Sodium hyaluronate aqueous solution products such as Hyalgan^{®}, ARTZ^{®}, Euflexxa^{®}, and ORTHOVISC^{®} have a molecular weight of about 500,000 to 3,600,000 Da and are said to exhibit pain-relieving effects of about 3 months in 3 replicates of 2 ml each at a time, and about 6 months in 5 replicates of 2 ml each at a time. Commercially available products in the form of cross-linked hyaluronic acid include Synvisc^{®}, Synvisc-one^{®}, Durolane^{®}, Gel-One^{®}, and MONOVISC^{®}, and these are products that increase the molecular weight of hyaluronic acid through crosslinking or protect the site where hyaluronic acid is decomposed by enzymes to increase the duration of the effect.

Patent Document 1 discloses a method including reacting hyaluronic acid (HA) with divinyl sulfone (DVS) in an aqueous alkali solution to form a cross-linked hyaluronic acid gel, and controlling the swelling ratio and the degree of crosslinking of the gel according to various conditions (polymer/DVS ratio, molecular weight and concentration of hyaluronic acid) during the reaction. The crosslinking reaction is carried out at pH 9 and 20 °C at a ratio of 15:1 to 1:5 by weight of HA/DVS with a molecular weight of hyaluronic acid of 50,000 to 8,000,000 Da and a concentration of 1 to 8%. This low reaction temperature is due to the rapid decomposition of HA at high temperatures and in alkaline solution which decreases the molecular weight, thereby reducing the effect on the properties of the cross-linked gel. Synvisc (Synvisc^{®}, Synvisc-one^{®}), a crosslinked hyaluronic acid gel injection product by the invention, is commercially available.

In addition, Patent Document 2 discloses a method for preparing a biocompatible polysaccharide gel composition, wherein the method includes reacting hyaluronic acid with 1,4-butanediol diglycidyl ether (BDDE) containing an epoxy functional group as a polyfunctional cross-linking agent to prepare a hyaluronic acid hydrogel having viscoelasticity. After primary crosslinking with a 0.2% crosslinking agent and 10% hyaluronic acid for ether formation at pH 9, pH is corrected to 2 to 6 by adding acetic acid to form a gel by a secondary reaction for ester formation. Durolane^{®}, a crosslinked hyaluronic acid product of a high molecular weight (9,000,000 Da), is commercially available using the technology of this invention.

Patent Document 3 discloses a method of preparing a hyaluronic acid hydrogel that forms a cyclobutane ring by UV after synthesizing a photoreactive hyaluronic acid derivative with cinnamic acid. The dynamic viscoelasticity values measured by a rheometer under a 10 Hz condition shows that a storage modulus (G'; elasticity) is 50 to 1,500 Pa and a loss modulus (G"; viscosity) is 10 to 300 Pa, so that the viscoelasticity is superior to that of the existing products. GEL-ONE^{®}, which is commercially available using the technology of this invention is said to have increased persistence in vivo because the amination of a carboxy group of hyaluronic acid, which is a recognition site of a hyaluronic acid degrading enzyme, is made and a degradation rate by hyaluronic acid is low compared to other commercially available products.

As such, since hyaluronic acid has a short half-life (persistence in the body) of only a few hours after application into the body, in order to increase the half-life and persistence, a method of increasing the dose of the composition, the concentration and molecular weight of hyaluronic acid has been attempted. However, when the hyaluronic acid is injected intraarticularly using a syringe due to increased viscosity, the injection force of the composition increases, making it difficult for it to infuse into the tissue, which causes pain and puts a burden on the patient and operator. In addition, the cross-linked hyaluronic acid form has increased in vivo persistence compared to un-crosslinked hyaluronic acid, but is still degraded within 6 months and has low biopersistence.

In order to solve the above problem, in the present invention, a hydrogel composition containing a 6-arm PEG derivative and whose physical properties are converted from a sol to a gel within 30 minutes over time is administered intraarticularly. The hydrogel composition can be injected at a low viscosity by administration in a sol state and lowers the injection force, and can be converted in physical properties to a gel state within 30 minutes after administration.

### [Related Art Document]

### [Patent Document]

(Patent Document 1) US Patent No. 4,582,865
(Patent Document 2) US Patent No. 5,827,937
(Patent Document 3) US Patent No. 6,031,017

### [Non-Patent Document]

(Non-Patent Document 1) Advancing Visco supplementation. 2007. Dr Ting Choon Meng
(Non-Patent Document 2) Disorders of the knee 2nd ed. JB Lippincott; 1982

### [Technical Problem]

The present invention is directed to providing a PEG hydrogel composition that is converted in physical properties from a sol to a gel within 30 minutes over time.

### [Technical Solution]

The present invention provides an injection including: a first solution including a first polyethylene glycol derivative having an electrophilic reactive group; and a second solution including a second polyethylene glycol derivative having a nucleophilic reactive group, wherein the first polyethylene glycol derivative includes 6-arm polyethylene glycol-N-hydroxysuccinimide (6-arm PEG-NHS) and/or 8-arm polyethylene glycol-N-hydroxysuccinimide (8-arm PEG-NHS), the second polyethylene glycol derivative includes 6-arm polyethylene glycol-thiol (6-arm PEG-SH) and/or 8-arm polyethylene glycol-thiol (8-arm PEG-SH), and the first solution and the second solution form a hydrogel through a sol-gel reaction upon mixing.

In addition, the present invention provides a kit for an injection including: a first solution set in which a first polyethylene glycol derivative powder having an electrophilic reactive group and a buffer solution of pH 4 to 9 are separately stored; and a second solution set in which a second polyethylene glycol derivative powder having a nucleophilic reactive group and a buffer solution of pH 4 to 9 are separately stored, wherein the first polyethylene glycol derivative includes 6-arm polyethylene glycol-N-hydroxysuccinimide (6-arm PEG-NHS) and/or 8-arm polyethylene glycol-N-hydroxysuccinimide (8-arm PEG-NHS), the second polyethylene glycol derivative includes 6-arm polyethylene glycol-thiol (6-arm PEG-SH) and/or 8-arm polyethylene glycol-thiol (8-arm PEG-SH), and a first solution and a second solution form a hydrogel through a sol-gel reaction upon mixing.

### [Effects of the Invention]

The present invention provides a PEG hydrogel that is converted in physical properties from a sol to a gel within 30 minutes over time.

In the present invention, two types of PEG derivatives are included as a main component of the hydrogel, and when each of the PEG derivatives is dissolved in a buffer solution, and then the two solutions are mixed, it can be converted in physical properties from a sol state to a gel state within 30 minutes. The PEG hydrogel is easily injected into the body in a liquid (sol) state, and after injection, the viscosity and elasticity of the composition increase and it is converted in physical properties to a gel form, so that it can be applied as a viscosupplementation for osteoarthritis pain relief, etc.

### [Brief Description of the Drawings]

FIG. 1 shows the gelation time of a hydrogel according to pH.
FIG. 2 shows the gelation time of the hydrogel according to a concentration of a 6-arm PEG derivative.
FIGS. 3 and 4 show viscoelasticity measurement results of the hydrogel.
FIG. 5 shows the measurement results of the joint pain relief effect of the hydrogel.

### [Best Mode]

The present invention relates to an injection which includes: a first solution including a first polyethylene glycol derivative having an electrophilic reactive group; and a second solution including a second polyethylene glycol derivative having a nucleophilic reactive group, wherein the first polyethylene glycol derivative includes 6-arm polyethylene glycol-N-hydroxysuccinimide (6-arm PEG-NHS) and/or 8-arm polyethylene glycol-N-hydroxysuccinimide (8-arm PEG-NHS), the second polyethylene glycol derivative includes 6-arm polyethylene glycol-thiol (6-arm PEG-SH) and/or 8-arm polyethylene glycol-thiol (8-arm PEG-SH), and the first solution and the second solution form a hydrogel through a sol-gel reaction upon mixing.

Hereinafter, the present invention will be described in more detail.

In the present invention, using two types of PEG derivatives, a hydrogel can be formed by crosslinking the two types of PEG derivatives.

Specifically, an injection according to the present invention includes a first solution and a second solution separately, the first solution includes a first polyethylene glycol derivative, and the second solution includes a second polyethylene glycol derivative. In this case, the first polyethylene glycol derivative includes 6-arm polyethylene glycol-N-hydroxysuccinimide (6-arm PEG-NHS) and/or 8-arm polyethylene glycol-N-hydroxysuccinimide (8-arm PEG-NHS), and the second polyethylene glycol derivative includes 6-arm polyethylene glycol-thiol (6-arm PEG-SH) and/or 8-arm polyethylene glycol-thiol (8-arm PEG-SH).

Hereinafter, the 6-arm PEG-NHS may be referred to as a first 6-arm PEG derivative, the 8-arm PEG-NHS may be referred to as a first 8-arm PEG derivative, the 6-arm PEG-SH may be referred to as a second 6-arm PEG derivative, and the 8-arm PEG-SH may be referred to as a second 8-arm PEG derivative. Also, hereinafter, the 6-arm PEG derivative may be understood to be both of the 6-arm PEG-NHS and the 6-arm PEG-SH, and the 8-arm PEG derivative may be understood to be both of the 8-arm PEG-NHS and the 8-arm PEG-SH.

In the present invention, the "6-arm PEG derivative" or "8-arm PEG derivative" is a derivative having 6 or 8 linear PEG groups from a core, respectively, and may have a form in which they are linked to each other from the core.

In the present invention, the first solution may include 6-arm polyethylene glycol-N-hydroxysuccinimide (6-arm PEG-NHS) and/or 8-arm polyethylene glycol-N-hydroxysuccinimide (8-arm PEG-NHS) having an electrophilic reactive group and a first solvent.

In the present invention, "6-arm polyethylene glycol-N-hydroxysuccinimide (6-arm PEG-NHS)" may have a structure in which each terminal portion of 6-arm PEG is substituted with N-hydroxysuccinimidyl (NHS). In addition, "8-arm polyethylene glycol-N-hydroxysuccinimide (8-arm PEG-NHS)" may have a structure in which each terminal portion of 8-arm PEG is substituted with N-hydroxysuccinimidyl (NHS). The NHS may react with a thiol group.

In one embodiment, the 6-arm PEG-NHS and/or the 8-arm PEG-NHS may be a compound represented by Chemical Formula 1 below.

[Chemical Formula 1] Core-[-(CH₂CH₂O)ₙ-(CH₂)ₘ₁-(L)ₚ-(CH₂)ₘ₂-R]_{q},

in Chemical Formula 1, L is a linker, and is 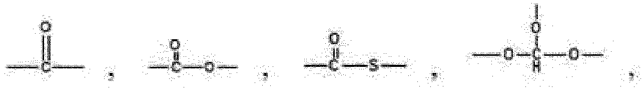 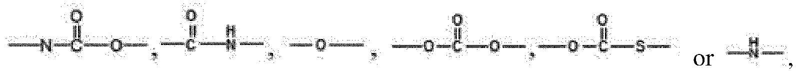
R is 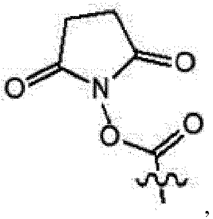
Core is 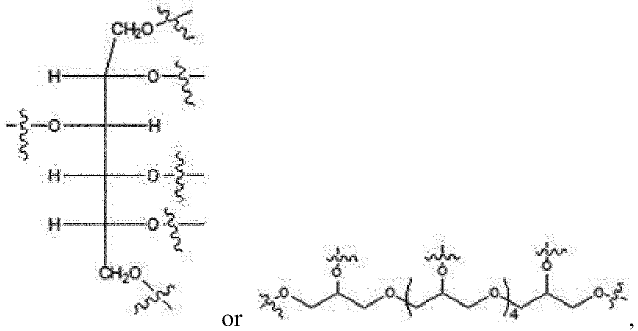
n is an integer of 10 to 2,500, m1 and m2 are each independently an integer of 0 to 3, p is an integer of 0 to 1, and q is an integer of 6 or 8.

When it has a 6-arm structure, Core may be 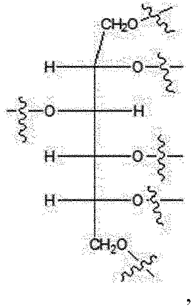 and when it has an 8-arm structure, Core may be

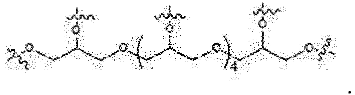

Specifically, the first polyethylene glycol derivative may be 6-arm PEG-NHS, wherein L may be -(C=O)-, R may be 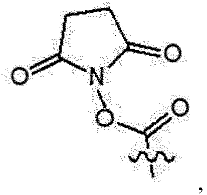 and Core may be 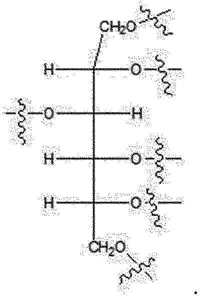 Also, n may be an integer of 20 to 400, m1 may be 0, m2 may be an integer of 1 to 3, p may be the integer 1, and q may be the integer 6.

In one embodiment, the 6-arm PEG-NHS may be a compound represented by Chemical Formula 3 below. 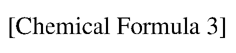 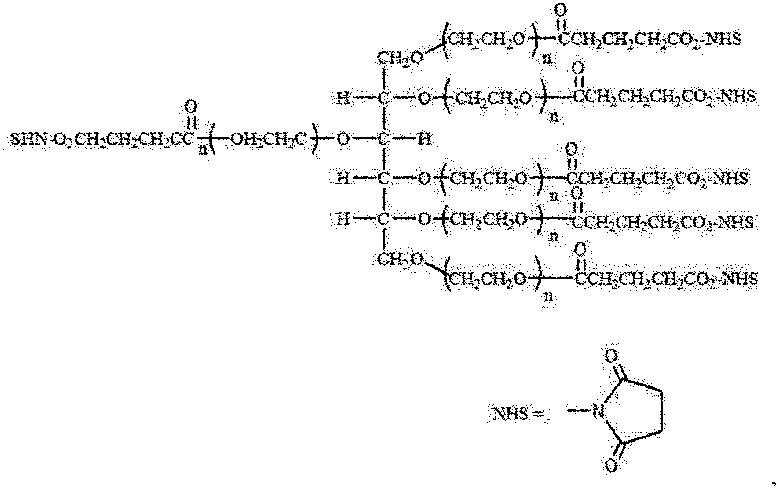 in Chemical Formula 3, n may be an integer of 20 to 400.

In the present invention, the second solution may include 6-arm polyethylene glycol-thiol (6-arm PEG-SH) and/or 8-arm polyethylene glycol-thiol (8-arm PEG-SH) having a nucleophilic reactive group and a second solvent.

In the present invention, "6-arm polyethylene glycol-thiol (6-arm PEG-SH)" may have a structure in which each terminal portion of 6-arm PEG is substituted with a thiol. In addition, "8-arm polyethylene glycol-thiol (8-arm PEG-SH)" may have a structure in which each terminal portion of 8-arm PEG is substituted with a thiol. The thiol can react with NHS.

In one embodiment, 6-arm PEG-SH and/or 8-arm PEG-SH may be a compound represented by Chemical Formula 2 below.

[Chemical Formula 2] Core-[-(CH₂CH₂O)ₙ-(CH₂)ₘ₁-(L)ₚ-(CH₂)ₘ₂-R]_{q},

in Chemical Formula 2, L is a linker, and is 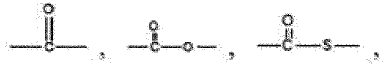 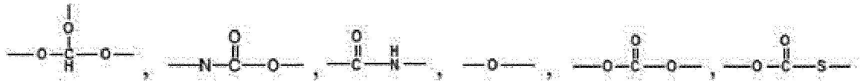 or 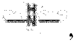
R is a thiol (SH) group,
Core is 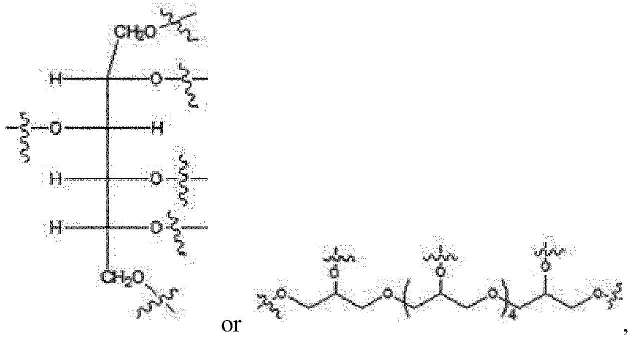
n is an integer of 10 to 2,500, m1 and m2 are each independently an integer of 0 to 3, p is an integer of 0 to 1, and q is an integer of 6 or 8.

When it has a 6-arm structure, Core may be 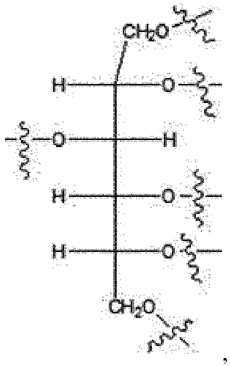 and when it has an 8-arm structure, Core may be

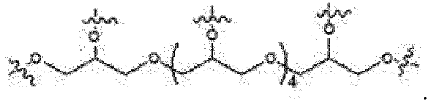

Specifically, the second polyethylene glycol derivative may be 6-arm PEG-SH, R may be a thiol group, and Core may be 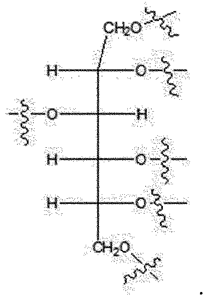 Also, n may be an integer of 20 to 400, m1 may be 0, m2 may be an integer of 1 to 3, p may be 0, and q may be the integer 6.

In one embodiment, the 6-arm PEG-SH may be a compound represented by Chemical Formula 4 below. 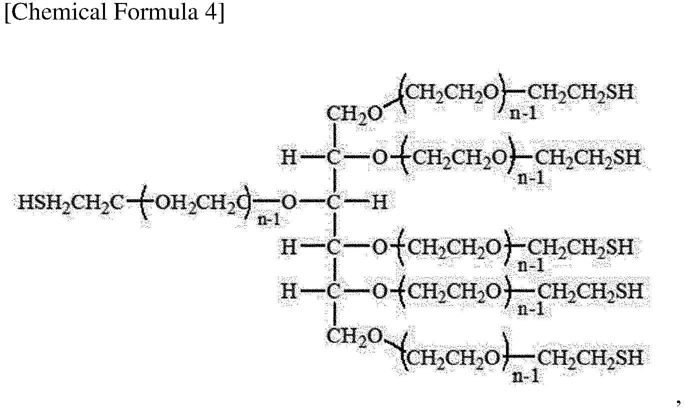 in Chemical Formula 4, n may be an integer of 20 to 400.

In the present invention, the molecular weight of the first polyethylene glycol derivative may be 5,000 to 100,000 Da, and the molecular weight of the second polyethylene glycol derivative may be 5,000 to 100,000 Da. Structural and physical properties of the prepared hydrogel can be controlled by controlling the molecular weight. The larger the molecular weight, the coarser the structure of the hydrogel, and the smaller the molecular weight, the finer the structure.

In the present invention, each of the first solvent and the second solvent may be a buffer solution. That is, the first polyethylene glycol derivative and the second polyethylene glycol derivative may each be included in the buffer solution. A phosphate buffer or physiological saline may be used as the buffer solution. The same solvent may be used as the first solvent and the second solvent.

In one embodiment, the pH of the first solution may be 4 to 9, and the pH of the second solution may be 4 to 9.

Further, in one embodiment, the concentration of the first polyethylene glycol derivative in the first solution may be 1 to 10% (w/v), and the concentration of the second polyethylene glycol derivative in the second solution may be 1 to 10% (w/v). As the concentration of the first polyethylene glycol derivative increases and the pH of the solution during the reaction is closer to basicity, the gelation time required for the formation of the hydrogel may be shortened. That is, when the concentration is less than 1%, the physical properties become close to sol, and when the concentration exceeds 10%, since it forms a hard gel and shows the physical properties of the broken form, it is not suitable for use in materials requiring viscoelasticity.

In the present invention, the first solution and the second solution may be mixed immediately before injection and used, and the first solution and the second solution may form a hydrogel within 30 minutes through a sol-gel reaction when mixed. That is, the mixture of the first solution and the second solution may form a hydrogel after injection.

In one embodiment, the first polyethylene glycol derivative having an electrophilic reactive group and the second polyethylene glycol derivative having a nucleophilic reactive group may be mixed in a weight ratio of 1:0.5 to 1:2 or 1:0.7 to 1:1.1.

The hydrogel is defined as structures of natural or synthetic derivatives that swell in aqueous solution but do not dissolve. In addition, it has many advantages that can be applied to the field of biomedicine. That is, it can absorb and encapsulate an aqueous solution, so that it is similar to a living tissue and has permeability to low-molecular substances such as oxygen, nutrients, and metabolites. In addition, since the surface structure of the swollen hydrogel is soft, it is possible to reduce irritation due to friction with surrounding cells or tissues in vivo. The present invention can provide an arthritis injection including two types of PEG derivatives, which is long-lasting in vivo and has high biocompatibility. The injection of the present invention lasts for a long time when administered once to a joint (cartilage cavity), so that a long-lasting analgesic effect and a protective effect on articular cartilage can be obtained. In addition, two types of PEG derivatives, which are biocompatible polymers, are used and react with each other in a neutral or basic aqueous solution, and therefore it can be converted in physical properties from a sol to a gel within 30 minutes of the reaction through cross-linking and covalent bonding between them, and the hydrogel can be formed.

That is, when the first solution and the second solution are mixed, the first polyethylene glycol derivative having an electrophilic reactive group and the second polyethylene glycol derivative having a nucleophilic reactive group may form a covalent bond. Specifically, a thiol group of the second polyethylene glycol derivative having a thiol group (SH) and NHS of the first polyethylene glycol derivative having NHS form a covalent bond to form a hydrogel (Scheme 1).

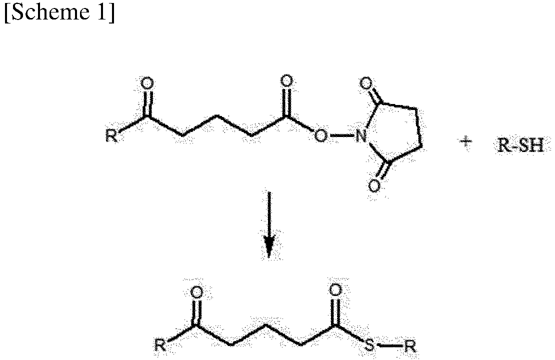

In the present invention, the pH of the first solution and the pH of the second solution may be different from each other. In a solution having the same pH, there is a risk of clogging the injection needle during injection due to rapid gelation, so that the gelation time can be controlled by using buffer solutions of different pH conditions.

In the present invention, when 6-arm PEG-NHS is used as the first polyethylene glycol derivative, 6-arm PEG-SH may be used as the second polyethylene glycol derivative. In addition, when 8-arm PEG-NHS is used as the first polyethylene glycol derivative, 8-arm PEG-SH may be used as the second polyethylene glycol derivative.

In one embodiment, the structural and physical properties of the hydrogel may be controlled by the molecular weight, concentration, and reaction conditions of the first polyethylene glycol derivative and/or the second polyethylene glycol derivative.

The injection according to the present invention may be intraarticularly (intracartilaginously) administered, and a hydrogel may be formed after injection.

In one embodiment, when the injection, specifically, the mixture of the first solution and the second solution is injected, the viscosity (loss modulus, G") within 300 seconds may be 1 Pa or less.

In addition, in one embodiment, when the injection, specifically, the mixture of the first solution and the second solution is injected, the elasticity (storage modulus, G') after 2,000 seconds may be 1,000 Pa or more.

Elasticity and viscosity (G', G"; Pa) values may indicate a high viscosity (1,000 Pa or more) in a gel state where covalent bonds are formed at low viscosity (1 Pa or less) close to sol.

The injection according to the present invention can reduce the disadvantage of the existing products that cause pain due to high injection force when intraarticularly injected due to their high viscosity. In addition, by controlling the crosslinking time, it is easy to inject from a syringe with a low viscosity when injected, and after injection, two types of PEG derivatives can react to form a hydrogel with excellent viscoelasticity while slowly forming a covalent bond.

In addition, the present invention provides a kit for storage of the injection, that is, a kit for injection.

The injection kit according to the present invention may include: a first solution set in which a first polyethylene glycol derivative powder having an electrophilic reactive group and a buffer solution of pH 4 to 9 are separately stored; and a second solution set in which a second polyethylene glycol derivative powder having a nucleophilic reactive group and a buffer solution of pH 4 to 9 are separately stored.

In this case, the first polyethylene glycol derivative includes 6-arm polyethylene glycol-N-hydroxysuccinimide (6-arm PEG-NHS) and/or 8-arm polyethylene glycol-N-hydroxysuccinimide (8-arm PEG-NHS), and the second polyethylene glycol derivative includes 6-arm polyethylene glycol-thiol (6-arm PEG-SH) and/or 8-arm polyethylene glycol-thiol (8-arm PEG-SH), and when the first solution and the second solution are mixed, a hydrogel may be formed through a sol-gel reaction

In the kit, a first solution is prepared by dissolving the first polyethylene glycol derivative powder in the buffer solution of the first solution set immediately before use, a second solution is prepared by dissolving the second polyethylene glycol derivative powder in the buffer solution of the second solution set, and the first solution and the second solution may be used after being mixed.

Hereinafter, the present invention will be described in detail by way of examples. The following examples merely illustrate the present invention but do not limit the scope of the present invention. The examples are merely provided to complete the disclosure of the present invention and to fully illuminate the scope of the invention to those skilled in the art, and the invention is only defined by the scope of the claims.

### [Mode for Invention]

### Examples

### Preparation Example 1. Synthesis of 6-arm PEG-succinimidyl glutarate (6-arm PEG-SG)

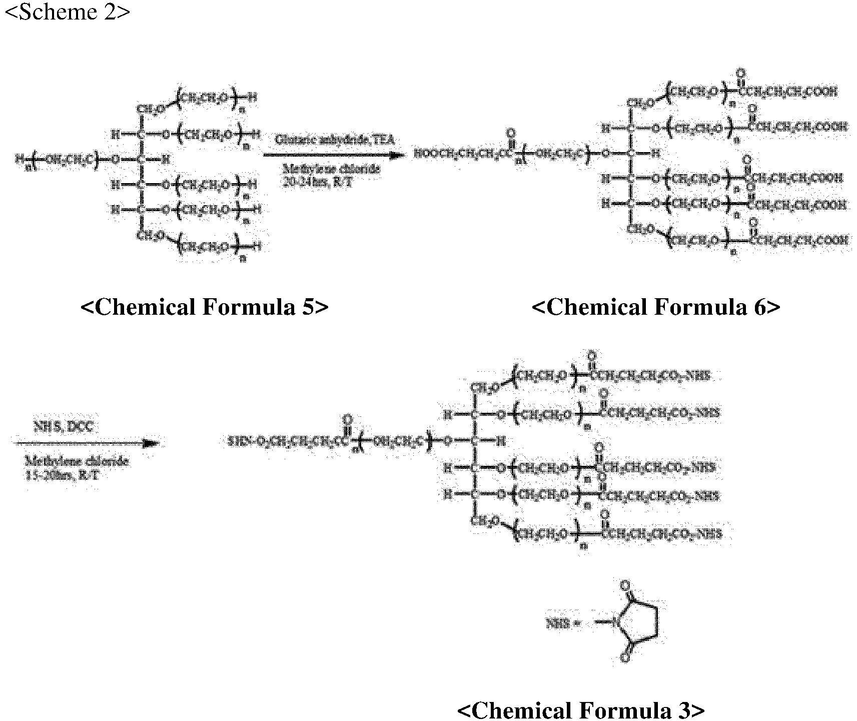

6-arm PEG-succinimidyl glutarate (6-arm PEG-SG) was synthesized according to Scheme 2 above.

After dissolving a compound of Chemical Formula 5 in methylene chloride at room temperature, triethylamine was added to prepare a reaction solution. After adding glutaric anhydride to the reaction solution, it was stirred at room temperature for 20 to 24 hours. The reaction solution was washed with a 14% ammonium chloride aqueous solution, and when layers were separated, a lower organic solution layer was collected. The aqueous solution layer was extracted using methylene chloride. The combined organic solution layer was precipitated in diethyl ether after moisture was removed using magnesium sulfate and the solvent was concentrated. The precipitate was filtered and dried under vacuum at room temperature for 24 hours to obtain a compound of Chemical Formula 6.

The compound of Chemical Formula 6 was dissolved in methylene chloride, and N-hydroxy succinimide (NHS) and dicyclohexyl carbodiimide (DCC) were added thereto to prepare a reaction solution. The reaction solution was stirred at room temperature for 15 to 20 hours. After the reaction, dicyclohexyl urea (DCU), which is a by-product, was filtered using a glass filter, and the filtered solution was precipitated in diethyl ether after concentrating the solvent. After filtering the precipitate, it was dissolved in ethyl acetate at 55±5 °C and recrystallized at 0 to 5 °C for 15 to 17 hours. The recrystallizate was filtered, washed 3 times with diethyl ether, and dried under vacuum at room temperature for 24 hours to obtain a compound (n=151) of Chemical Formula 3 having a weight-average molecular weight of 40,000.

### Preparation Example 2. Synthesis of 6-arm PEG-SH

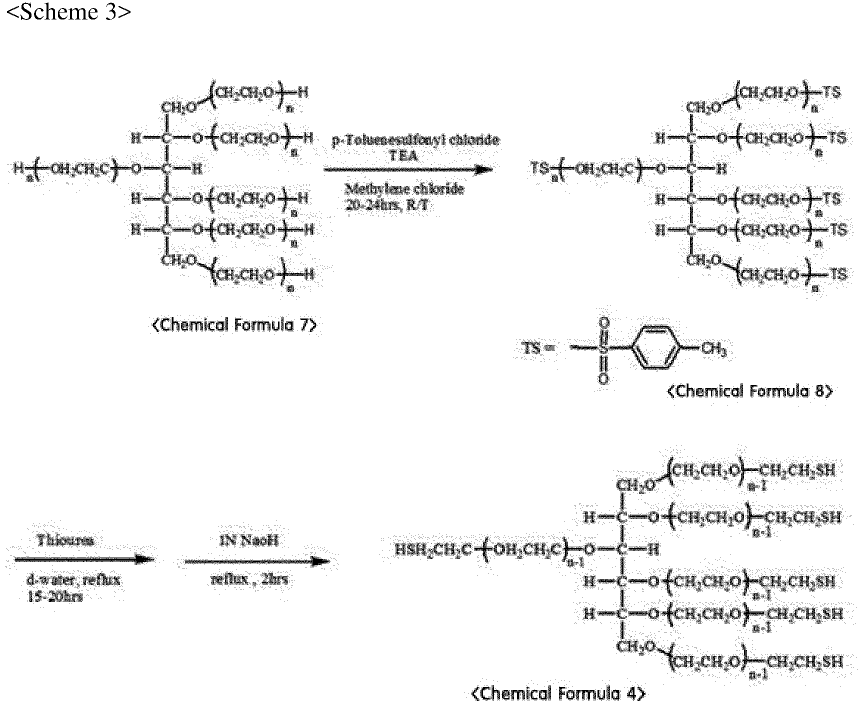

6-arm PEG-SH was synthesized according to Scheme 3 above.

After dissolving a compound of Chemical Formula 7 in methylene chloride at room temperature, triethylamine was added to prepare a reaction solution. P-toluenesulfonyl chloride was added to the reaction solution and stirred at room temperature for 20 to 24 hours. The reaction solution was washed with a 14% ammonium chloride aqueous solution, and when aqueous solution layers were separated, a lower organic solution layer was collected. The aqueous solution layer was extracted using methylene chloride. The combined organic solution layer was precipitated in diethyl ether after moisture was removed using magnesium sulfate and the solvent was concentrated. The precipitate was filtered and dried under vacuum at room temperature for 24 hours to obtain a compound of Chemical Formula 8.

After dissolving the compound of Chemical Formula 8 in water, thiourea was added to prepare a reaction solution. The reaction solution was stirred at reflux temperature for 15 to 20 hours. After that, a 1 N NaOH solution was added and stirred at reflux temperature for 2 hours. Methylene chloride was added to the reaction solution and extracted twice. The combined organic layer was precipitated in diethyl ether after moisture was removed using magnesium sulfate and the solvent was concentrated. The precipitate was filtered and dried under vacuum at room temperature for 24 hours to obtain a compound of Chemical Formula 4 (n=151) having a weight-average molecular weight of 40,000.

### Examples 1 to 5

The 6-arm PEG-SG prepared in Preparation Example 1 and the 6-arm PEG-SH prepared in Preparation Example 2 were added to a PBS buffer solution to prepare solutions 1 and 2, respectively, and the pH of the buffer solution was adjusted according to the conditions to prepare a hydrogel by the method of Table 1 below.

**[Table 1]**

| Classification | Solution 1 | Solution 2 |
|---|---|---|
| | 6-arm-PEG-SG-40k | 6-arm PEG-SH-40k |
| | 30 mg/ml Phosphate buffer | 30 mg/ml Phosphate buffer |
| Example 1 | pH4 | pH8 |
| Example 2 | pH5 | pH8 |
| Example 3 | pH6 | pH8 |
| Example 4 | pH7 | pH8 |
| Example 5 | pH8 | pH8 |

### Experimental Example 1. Measurement of gelation time according to pH of Solution 1

1 ml of each of the solutions 1 and 2 of Examples 1 to 5 was put in a glass test tube, and vortexed for 10 seconds, and then gelation time was confirmed at room temperature. At this time, the gelation time was measured when the glass test tube was turned over, there was no flow of the mixed solution, and it was fixed to the bottom of the test tube and hardened without movement. The test was repeated three times, and the average value and standard deviation were graphed.

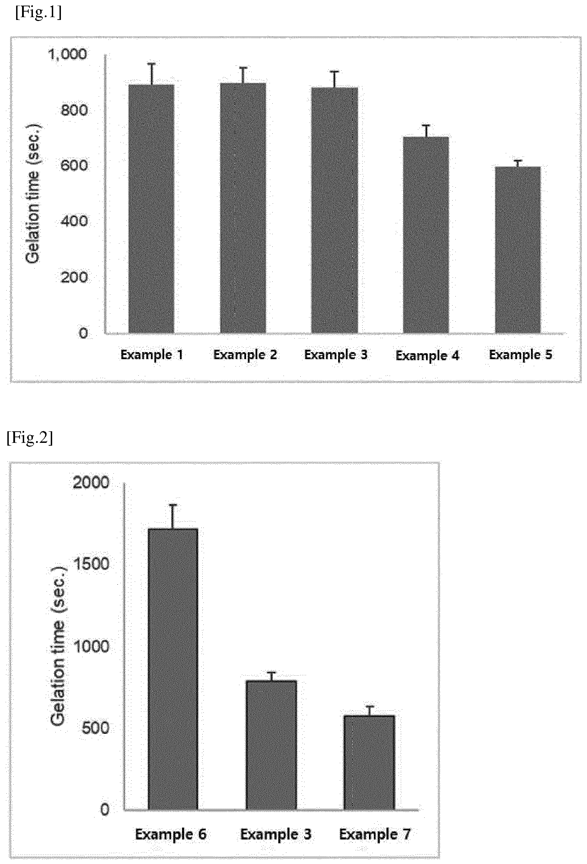

FIG. 1 shows the gelation time of a hydrogel according to pH.

As shown in FIG. 1, Examples 1 to 5 show a tendency for the gelation time to decrease according to the pH of the solution 1, and in particular, in Examples 3 to 5, it can be seen that the gelation time is greatly shortened as the pH of the solution 1 increases. The gelation time of Examples 1 to 3 was measured at about 15 minutes, that of Example 4 was measured in less than 12 minutes, and that of Example 5 was measured in less than 10 minutes.

### Examples 6 and 7

The 6-arm PEG-SG prepared in Preparation Example 1 and the 6-arm PEG-SH prepared in Preparation Example 2 were added to a PBS buffer solution to prepare solutions 1 and 2, respectively, and a hydrogel was prepared by the method of Table 2 below.

**[Table 2]**

| Classification | Solution 1 | Solution 2 |
|---|---|---|
| | 6-arm-PEG-SG-40k | 6-arm-PEG-SH-40k |
| | /ml phosphate buffer (pH6.0) | /ml phosphate buffer (pH8.0) |
| Example 3 | 30 mg | 30 mg |
| Example 6 | 20 mg | 20 mg |
| Example 7 | 40 mg | 40 mg |

### Experimental Example 2. Measurement of gelation time according to concentration of 6-arm PEG derivative

Gelation time was measured in the same manner as in Experimental Example 1 using the solutions 1 and 2 of Examples 3, 6 and 7.

FIG. 2 shows the gelation time of the hydrogel according to the concentration of the 6-arm PEG derivative.

As shown in FIG. 2, it can be seen that the gelation time is shortened as the concentration of the 6-arm PEG derivative increases.

### Experimental Example 3. Physical property test - Rheological test

"Viscosity" refers to viscosity, and "elasticity" refers to a force of something returning to its original state, that is, the force that a spring exerts to return to its original state when pulled. The combination of these two properties is called viscoelasticity. When a force is applied to an object, a flow phenomenon having both elasticity and viscosity occurs, and in general, a polymer solution has both viscosity and elasticity (restorative force), that is, viscoelasticity.

A hydrogel was prepared as in the examples using a 6-arm PEG derivative, and rheometer analysis was performed to measure the viscoelasticity of the hydrogel. Analysis was performed using DHR1 (Discovery hybrid Rheometer, TA Instruments Ltd., USA) equipment at 37 °C, using a 40 mm plate, and a rheological test was performed with a 0.5 mm gap, 1% strain, and a 2.5 Hz frequency oscillation mode.

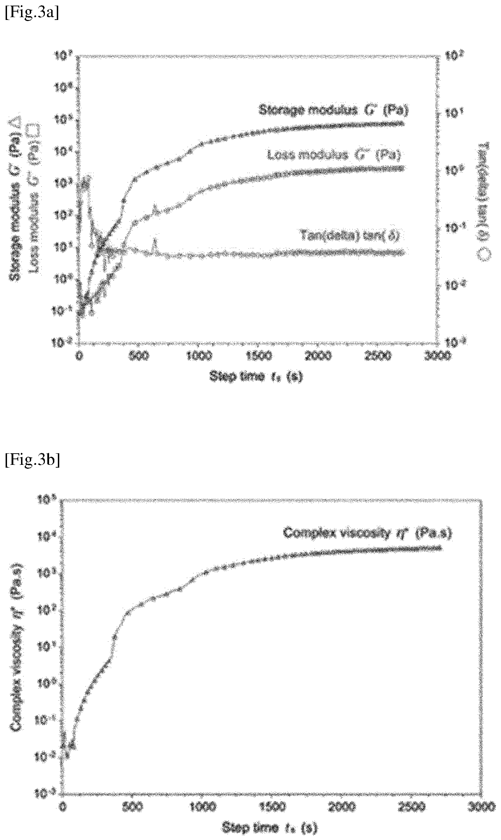

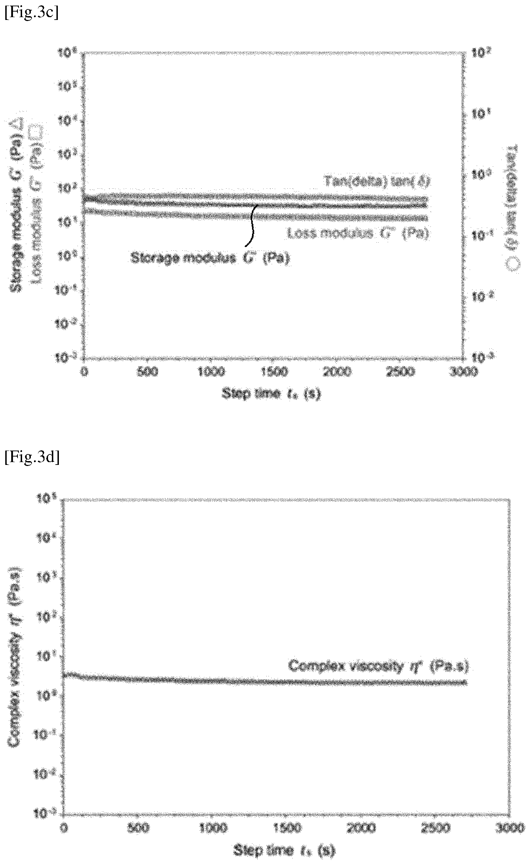

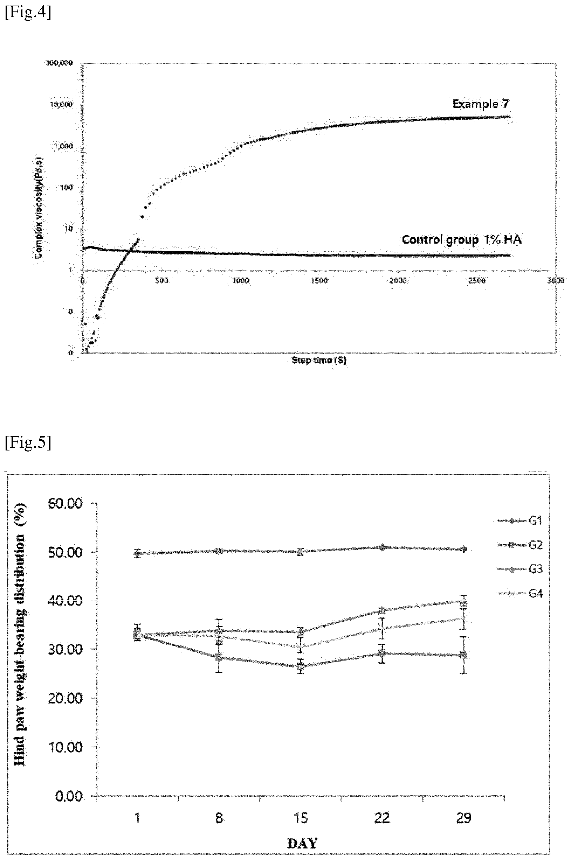

FIG. 3 and 4 show the viscoelasticity measurement results of the hydrogel.

In FIG. 3, G' is a numerical value indicating elasticity as a storage modulus or elastic modulus, and the higher the strength of the sample and the greater the resistance to deformation, the higher the value. G" is a numerical value indicating viscosity. Tan delta is G"/G', and the larger the value, the greater the viscosity than the elasticity. In addition, complex viscosity is a numerical value that reflects both viscosity and elasticity, and represents the degree of deformation with respect to external force.

(A) and (B) of FIG. 3 are the hydrogels prepared with the composition of Example 7, (C) and (D) show the rheometer analysis result of 1% hyaluronic acid (HA; high viscosity 3.3, 3,500,000 to 4,200,000 Da; Bioland) as a control group.

As shown in FIG. 3, the G' elasticity value of 1% hyaluronic acid as a control group was about 33 to 35 Pa, the G" viscosity value was 13 to 16 Pa, the tan delta value was 0.4, and the complex viscosity was 2.3 to 2.5 Pa. s. That is, 1% hyaluronic acid exhibited constant viscosity and elasticity that did not change with time.

On the other hand, in the case of the hydrogel of Example 7, the G' elasticity value in the first 6 seconds was 0.32 Pa, and the G" viscosity value was 0.09 Pa (the viscosity of water at 10 °C, 0.001 Pa), indicating low viscosity. However, as time passed, the viscosity and elasticity significantly increased, with G' of 10,000 Pa or more, G" of 1,000 Pa or more, tan delta value of 0.03, and complex viscosity of 2,000 Pa·s at 1500 seconds (25 minutes).

In addition, FIG. 4 shows the results of comparing the complex viscosity of the hydrogel of Example 7 and 1% HA as a control group.

As shown in FIG. 4, compared to the constant value of 1% HA, the hydrogel of Example 7 showed lower viscoelasticity than the control group within about 300 seconds of the initial analysis, but it was confirmed that it showed higher viscoelasticity after about 300 seconds

### Experimental Example 4. Confirmation of joint-pain-reducing effect

Using a rat MIA arthritis induction model commonly used as an arthritis model, the arthritis pain-reducing efficacy of the injection of Example 7 and the positive control group was confirmed.

First, after hair removal around the right hind knee of the rat, 50 µl (60 mg/ml) of an osteoarthritis-inducing substance, MIA (Monosodium iodoacetate, Sigma Chemical Co. Ltd, Cat No. I9148), was injected into the knee joint cavity using a Hamilton syringe to induce osteoarthritis (Corinne guingamp et al., Mono-iodoacetate-induced experimental Osteoarthritis, Arthritis & Rheumatism, 1997, 40(9), 1670-1679, Kai Gong et al., Journal of the Formosan medical association, 2011, 110(3), 145-152).

One week after MIA injection, 50 µl of each of the injection of Example 7 and 1% (10 mg/ml) sodium hyaluronate as the positive control group, was injected into the knee joint cavity.

The joint-pain-reducing effect was evaluated by measuring the weight (g) of each of the left and right hind paws using an Incapacitance tester (Stoelting Co., Wood Dale, IL) as a foot weigher before osteoarthritis induction (week 0) and on weeks 1, 2, 3 and 4 after osteoarthritis induction and calculating the change in right hind paw weight distribution (HPWD, %) according to Equation 1 below. The paw weight was measured 3 times per animal.

The weight change rate on the right hind paw is a value expressed by calculating the percentage of the weight placed on the left hind paw due to pain in the right knee caused by arthritis in the right leg, and about 50% of the measured values are considered normal without arthritis. Right hind paw weight (%) = [right hind paw weight/(right hind paw weight + left hind paw weight)] x 100

The measured values were expressed as mean (%) ± standard error by calculating a ratio of the weight of the right hind paw to the weight of both hind paws.

The measurement results are shown in Table 3 and FIG. 5.

**[Table 3]**

| Group | Week 0 | Week 1 | Week 2 | Week 3 | Week 4 |
|---|---|---|---|---|---|
| Normal group (G1) | 49.65±0.86 ** | 50.18±0.44 ** | 50.01±0.58 ** | 50.92±0.29 ** | 50.52±0.25 ** |
| Excipient control group (G2) | 33.11±0.89 | 28.32±2.97 | 26.53±1.48 | 29.14±1.90 | 28.82±3.74 |
| Example 7 (G3) | 33.11±1.17 | 33.98±2.16 | 33.67±1.99 ** | 38.12±0.39 ** | 40.00±1.14 * |
| Positive control group (G4) | 33.10±1.40 | 32.73±1.64 | 30.45±1.08 * | 34.38±2.12 | 36.32±2.09 |

In Table 3, each value was expressed as mean±S.D, and parametric comparison was performed by One-Way ANOVA or Student' t-test ((N=7). *P<0.05, **P<0.01: excipient control group (G2)).

As shown in Table 3 and FIG. 5, in the case of the excipient control group (group without hydrogel treatment after arthritis induction, G2), the weight change rate carried on the right hind paw was kept low at 26-33% from week 1 to week 4, while in the injection (G3) of Example 7, the weight change rate carried on the right hind paw after administration of the test substance was recovered to 38% at week 3 and 40% at week 4, and thus a significant drug effect was observed. The weight ratio carried on the right hind paw of the positive control group (G4) also increased to 34% at week 3 and 36% at week 4, thereby indicating that the injection according to the present invention had a similar or better pain relief effect than the positive control group.

### [Industrial Applicability]

In the present invention, two types of PEG derivatives are included as a main component of the hydrogel, and when each of the PEG derivatives is dissolved in a buffer solution, and then the two solutions are mixed, it can be converted in physical properties from a sol state to a gel state within 30 minutes. The PEG hydrogel is easily injected into the body in a liquid (sol) state, and after injection, the viscosity and elasticity of the composition increase and it is converted in physical properties to a gel form, so that it can be applied as a viscosupplementation and the like for osteoarthritis pain relief.

## Claims

1. An injection comprising:
a first solution including a first polyethylene glycol derivative having an electrophilic reactive group; and
a second solution including a second polyethylene glycol derivative having a nucleophilic reactive group,
wherein the first polyethylene glycol derivative includes 6-arm polyethylene glycol-N-hydroxysuccinimide (6-arm PEG-NHS) and/or 8-arm polyethylene glycol-N-hydroxysuccinimide (8-arm PEG-NHS),
the second polyethylene glycol derivative includes 6-arm polyethylene glycol-thiol (6-arm PEG-SH) and/or 8-arm polyethylene glycol-thiol (8-arm PEG-SH), and
the first solution and the second solution form a hydrogel through a sol-gel reaction upon mixing.

2. The injection of claim 1, wherein the 6-arm polyethylene glycol-N-hydroxysuccinimide and/or the 8-arm polyethylene glycol-N-hydroxysuccinimide are a compound represented by the following Chemical Formula 1:
[Chemical Formula 1] Core-[-(CH₂CH₂O)ₙ-(CH₂)ₘ₁-(L)ₚ-(CH₂)ₘ₂-R]_{q},
in Chemical Formula 1, L is a linker, and is 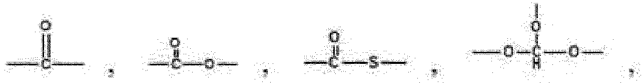 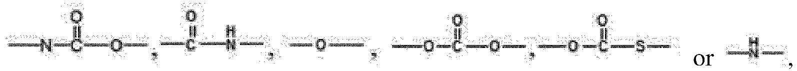
R is 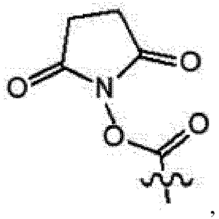
Core is 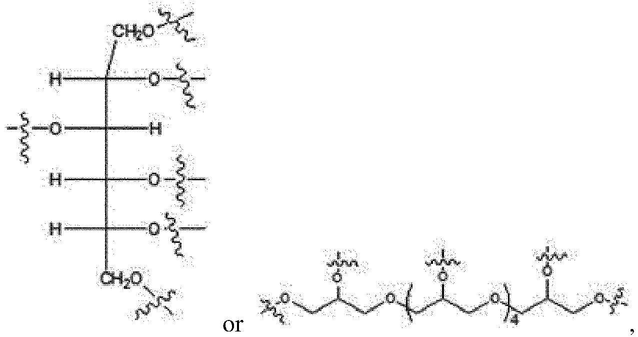
n is an integer of 10 to 2,500, m1 and m2 are each independently an integer of 0 to 3, p is an integer of 0 to 1, and q is an integer of 6 or 8.

3. The injection of claim 1, wherein the 6-arm polyethylene glycol-N-hydroxysuccinimide is a compound represented by the following Chemical Formula 3: 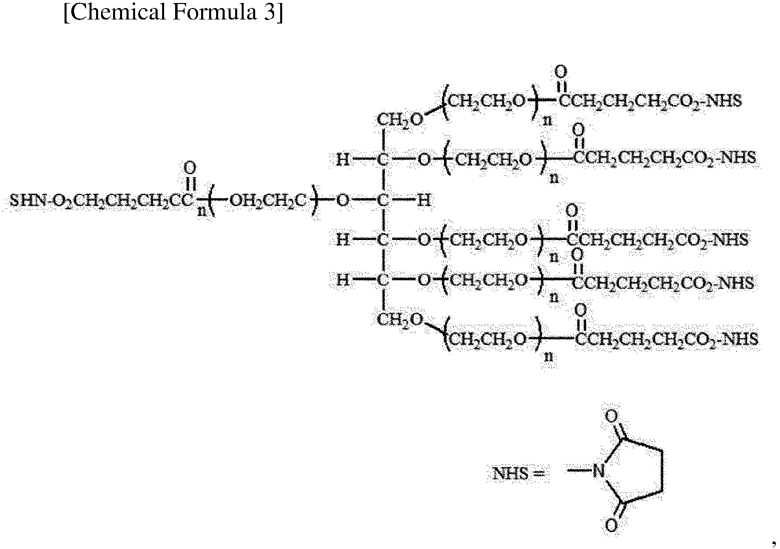 in Chemical Formula 3, n is an integer of 20 to 400.

4. The injection of claim 1, wherein the 6-arm polyethylene glycol-thiol and/or the 8-arm polyethylene glycol-thiol is a compound represented by the following Chemical Formula 2:
[Chemical Formula 2] Core-[-(CH₂CH₂O)ₙ-(CH₂)ₘ₁-(L)ₚ-(CH₂)ₘ₂-R]_{q}
in Chemical Formula 2, L is a linker, and is 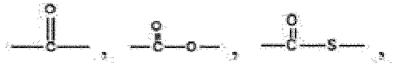 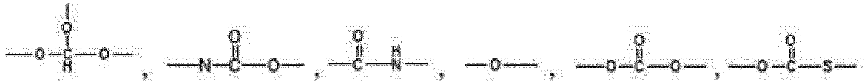 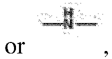
R is a thiol (SH) group,
Core is 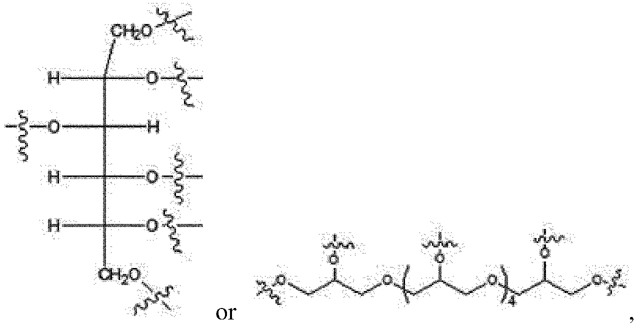
n is an integer of 10 to 2,500, m1 and m2 are each independently an integer of 0 to 3, p is an integer of 0 to 1, and q is an integer of 6 or 8.

5. The injection of claim 1, wherein the 6-arm polyethylene glycol-thiol is a compound represented by the following Chemical Formula 4: 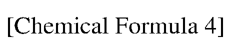 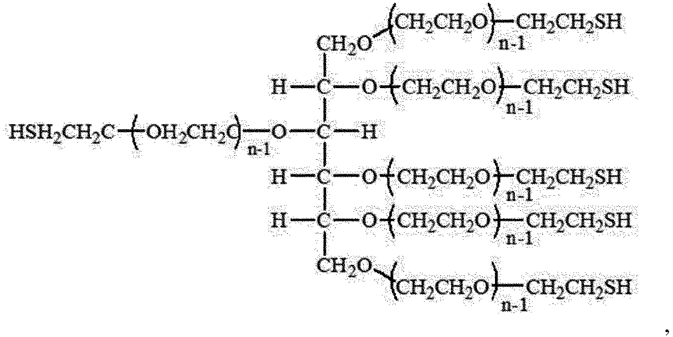 in Chemical Formula 4, n is an integer of 20 to 400.

6. The injection of claim 1, wherein a molecular weight of the first polyethylene glycol derivative in the first solution is 5,000 to 100,000 Da, and
a molecular weight of the second polyethylene glycol derivative in the second solution is 5,000 to 100,000 Da.

7. The injection of claim 1, wherein in the first solution and the second solution, solvents are each a buffer solution,
pH of the first solution is 4 to 9, and
pH of the second solution is 4 to 9.

8. The injection of claim 1, wherein a concentration of the first polyethylene glycol derivative in the first solution is 1 to 10% (w/v), and
a concentration of the second polyethylene glycol derivative in the second solution is 1 to 10% (w/v).

9. The injection of claim 1, wherein the first solution and the second solution are mixed immediately before injection, and
a mixture of the first solution and the second solution forms a hydrogel after injection.

10. The injection of claim 1, wherein a weight ratio of the first polyethylene glycol derivative and the second polyethylene glycol derivative is 1:0.5 to 1:2.

11. The injection of claim 9, wherein, when the first solution and the second solution are mixed, the first polyethylene glycol derivative and the second polyethylene glycol derivative form a covalent bond.

12. The injection of claim 9, wherein, when the mixture of the first solution and the second solution is injected, viscosity (loss modulus, G") within 300 seconds is 1 Pa or less.

13. The injection of claim 9, wherein, when the mixture of the first solution and the second solution is injected, elasticity (storage modulus, G') after 2,000 seconds is 1,000 Pa or more.

14. The injection of claim 1, which is intraarticular injection.

15. The injection of claim 1, which has an effect of suppressing pain caused by an arthritic disease or protecting articular cartilage.

16. A kit for an injection, comprising:
a first solution set in which a first polyethylene glycol derivative powder having an electrophilic reactive group and a buffer solution of pH 4 to 9 are separately stored; and
a second solution set in which a second polyethylene glycol derivative powder having a nucleophilic reactive group and a buffer solution of pH 4 to 9 are separately stored,
wherein the first polyethylene glycol derivative includes 6-arm polyethylene glycol-N-hydroxysuccinimide (6-arm PEG-NHS) and/or 8-arm polyethylene glycol-N-hydroxysuccinimide (8-arm PEG-NHS),
the second polyethylene glycol derivative includes 6-arm polyethylene glycol-thiol (6-arm PEG-SH) and/or 8-arm polyethylene glycol-thiol (8-arm PEG-SH), and
a first solution and a second solution form a hydrogel through a sol-gel reaction upon mixing.

17. The kit of claim 16, wherein a first solution is prepared by dissolving the first polyethylene glycol derivative powder in the buffer solution of the first solution set immediately before use,
a second solution is prepared by dissolving the second polyethylene glycol derivative powder in the buffer solution of the second solution set, and
the first solution and the second solution are mixed.
